# EUROPEAN PATENT APPLICATION

(11) **EP 3 109 840 A1**
(43) Date of publication of application: **28.12.2016**
(21) Application number: 15382339.8
(22) Date of filing: 26.06.2015
(51) Int. Cl.: G08B 21/24, G06F 19/00, G08B 13/196, G06K 9/00

(54) **HYGIENE COMPLIANCE MONITORING SYSTEM**

(71) Applicant: Fundació Institut de Recerca Biomédica de Bellvitge (IDIBELL), 08907 Barcelona (ES); Salutis Technological Solutions, S.L (SALUTISTECH), 08014 Barcelona (ES)
(72) Inventor: REGUÉ MORRERES, Joan Ramon, 08014 Barcelona (ES); GRAELLS I CASTELLÁ, Simó, 08014 Barcelona (ES); FRAILE ALGECIRAS, Enric, 08014 Barcelona (ES); GAVALDÀ MESTRE, Laura, 08907 L'Hospitalet de Llobregat (ES); CASADO GARCÍA, María Antonia, 08907 L'Hospitalet de Llobregat (ES); SORIANO FERRÍN, Ana María, 08840 Viladecans (ES)
(74) Representative: Hoffmann Eitle

(57) **Abstract**

The present invention relates to a controller in particular for a hand hygiene compliance monitoring system. The controller is preferably configured to detect contact between a HCW and a patient. The controller is configured to receive first distance information to determine first position information representing a position of at least one person and person identification information for identifying the at least one person. The controller is configured to receive an image and to determine second position information relating to the position of an imaged person based on the second distance information. The controller is configured to compare the first and the second position information and to identify the at least one person at the position represented by the first position information with the at least one person at the position represented by the second position information when the first position information matches the second position information.

## Description

### 1. Field of the invention

The present invention relates in particular to monitoring of compliance with hygiene protocols e.g. for hospitals or other medical facilities and provides a system, a controller and a method in particular for monitoring hygiene compliance. The invention is applicable to facilities where compliance with hygiene protocols is of importance as for example for hospital facilities and the like.

### 2. Technical background

Compliance with hygiene protocols such as compliance with the "Five moments for hand hygiene" developed by the World Health Organization is crucial in facilities where many people potentially come into contact, and in particular in medical facilities such as hospitals, to avoid spreading of diseases. Due to the growing awareness of the importance of compliance with hygiene protocols, corresponding monitoring systems have been developed during the recent years. Systems have been developed using e.g. standard video cameras for monitoring in particular health care workers ("HCWs") when working in patient rooms. Further systems make use of suitable sensors to detect HCWs using dispensers for cleaning agents such as soap or sterilizing agent dispensers.

An example is described in document WO 2013/106440 A1 which discloses a system based on a hand dispenser mounted close to a patient bed. When a HCW enters the vicinity of the dispenser and the patient bed, an alert is automatically generated. In response to the alert, the HCW enters a code corresponding to one of the WHO's Moments.

A further development in the present field provides a system employing an IR camera which can communicate with corresponding batches provided at HCWs. This system is described in the international publication WO 2010/151802 A2 which discloses a system for determining compliance with hand hygiene requirements in hospitals. The system according to this document is provided with a camera which allows for acquiring image frames (visible and IR) and performing video analysis. Using this camera it is suggested to monitor motions of a HCW within a patient room and to detect entry of the HCW into a contact zone around the patient bed.

The present invention has been made to further improve the prior art. It is a particular object of the present invention to provide a system preferably for hygiene compliance monitoring capable of determining and following a position of a person such as a HCW within a monitoring area such as a room in a hospital or other medical facilities with improved precision. It is a further object of the present invention to provide a system preferably for hygiene compliance monitoring capable of monitoring compliance with hygiene protocols such as the "Five moments for hand hygiene" defined by the WHO in an automatized manner.

These and other objects which become apparent when reading the following description are solved by a controller according to claim 1, a system according to claim 11, and by a method according to claim 15.

### 3. Summary of the invention

According to the invention, a controller is provided. In a preferred embodiment, the controller is a controller for a hygiene compliance monitoring system. Preferably, the controller is used specifically for monitoring hand hygiene. The controller comprises first distance information receiving means configured to receive first distance information acquired by at least one first transceiver. Preferably, the first distance information receiving means are provided at a predefined position within a monitoring area. The monitoring area may be a room such as a hospital room or may be defined by a transmission range of the used transceivers. Thereby, the first distance information represents a distance between the at least one first transceiver and at least one second transceiver provided at or for at least one person.

In a preferred embodiment, the at least one first transceiver is provided at a predefined position within a monitoring area, which in a preferred embodiment is a room such as a hospital or patient room. Preferably, the at least one first transceiver is provided at a wall of the room.

Preferably, the first distance information is distance information acquired by at least two, preferably by at least three, most preferably by at least four first transceivers provided at respective predefined positions within the monitoring area representing relative distances between each of the first transceivers and a second transceiver provided at a person within the monitoring area. In a preferred embodiment, the monitoring area is defined by a transmission range of the at least one first and/or the at least one second transceiver. This transmission range may be a maximum transmission range or may be limited e.g. by walls of a room such as walls of a hospital room wherein the hygiene compliance system is applied.

Further, in a preferred embodiment the first transceiver and the second transceiver are radio frequency transceivers (RF), for example the first transceiver can be an RF listener, i.e. receiver, and the second transceiver can be an RF badge or tag provided at a person. The second transceiver can thus for example be an RF tag attached to the clothing of a person such as a health care worker (HCW) or a patient or can be provided in form of an RF card carried by a person. Alternatively, the first and second transceivers can be different suitable transceivers such as for example infrared (IR) or ultrasound transceivers. The above mentioned monitoring area may be defined by a transmission range of the used transceivers.

For example four first transceivers such as four RF listeners can be distributed within a room, for example be provided at walls of the room. These first transceivers can then communicate with a second transceiver such as an RF badge provided at a person which is present in the monitoring area such as the room. Preferably, the first distance information is calculated based on the time needed to communicate a signal between the first and the second transceiver. For example, with bidirectional communication between first transceiver and second transceiver, the first transceiver may send a signal or message to the second transceiver. The second transceiver may reply after a prefixed time interval to the first transceiver. The total time is twice the time of flight plus the prefixed time. Based thereon, the first transceiver can calculate the distance between first and second transceiver as first distance information. The first distance information can be similarly calculated based on the time of one directional communication.

Alternatively, in a different embodiment the second transceiver may send a signal or a message to two or more synchronized first transceivers. Based on the difference in time of arrival at the first transceivers as first distance information, the controller can be configured to calculate a position of the second transceiver e.g. using a multilateration algorithm.

In the same way as described above, it is possible that further first distance information is determined for a case of more than one person. For example, in the case of two persons, two second transceivers may communicate with the at least one first transceiver and the controller then receives two times first distance information.

According to the invention, the controller is configured to determine first position information representing a position of the at least one person based on the first distance information. In other words, using e.g. the system of RF transceivers, the controller can determine a position of a person provided with the second transceiver. To this end, different algorithms can be implemented to perform the corresponding position calculation such as trilateration, multilateration or the like.

Further, according to the invention, the controller comprises person identification information receiving means configured to receive person identification information from a person ID transceiver for identifying the at least one person. Preferably, the identification information is received from the second transceiver. For example, each second transceiver such as an RF badge or tag can be provided with unique person identification information to identify the person provided with the second transceiver.

As a result, due to the communication of the at least one first transceiver and the second transceiver provided at a person, the controller is provided with the identity of the person e.g. within the monitoring area in addition to the position of the person. Similarly, in the case of multiple persons, the controller can thus be provided with identification of the persons in addition to their location e.g. within a patient room. Thus, the controller may e.g. determine position of a HCW in a patient room as well as the position of the patient. In addition, using the identification information, the controller is provided with the corresponding ID at the determined position. A particularly high spatial resolution can be achieved by using RF transceivers as first and second transceivers which may be on the order of only a few centimeters.

According to the invention, the controller further comprises two-dimensional image data receiving means configured to receive data of a two-dimensional image acquired by an imaging means. Data of a two-dimensional image may be digital data of a two-dimensional image acquired by a two-dimensional digital camera or data acquired by a stereoscopic camera using more than one objective lens to acquire more than one image to be combined into a two-dimensional image including 3D information. The imaging means can be adapted for imaging using visible light or infrared light. The imaging means can for example be provided at a wall of a patient room capturing images of the corresponding view of the room such that objects and persons present in the room are imaged within respective portions of the two-dimensional image.

According to the invention, the controller comprises a second distance information receiving means configured to receive second distance information representing at least one distance between the at least one person and the imaging means, whereby the at least one person is imaged by the imaging means. The second distance information may be received by the second distance information receiving means through calculation from data acquired by a 3D-camera, e.g. by a stereoscopic camera. The calculation may be e.g. performed at the controller or at the stereoscopic camera or by a separate calculation means.

The second distance information may further be provided by a second distance information acquiring means which may be a separate, dedicated system provided in addition to the imaging means. Such system may measure a distance between the imaging means for example by measuring a time of flight of an electromagnetic signal such as a light signal and determine the distance between the object or person and the imaging means based thereon. The second distance information acquiring means may be provided combined with the imaging means to form a 3D-camera.

In a preferred embodiment the second distance information is a two-dimensional array of distance information elements, and the data of the two-dimensional image is a two-dimensional array of image information elements, whereby each of the distance information elements has a one-to-one relation with a corresponding one of the image information elements and represents a distance between an object imaged within an image information element and the imaging means.

Thus, the second distance information can for example be a "depth image" corresponding to the two-dimensional image with portions of said depth image corresponding to portions of the two-dimensional image. The portions of the depth image then represent distances of objects imaged in the corresponding image portions with respect to the imaging means. Thereby, the distance information elements can be portions of the depth image being groups of pixels or individual pixels. Similarly, the distance information elements can be portions of the two-dimensional image represented by groups of pixels or individual pixels corresponding to the respective depth information elements.

According to the invention, the controller is further configured to determine second position information relating to the position of the at least one person imaged by the imaging means based on the second distance information.

For example, the controller may be configured to transform the second distance information into coordinates of the at least one person in an appropriately defined coordinate system provided for the monitoring area as the second position information. To this end, the controller can for example apply computer vision techniques to the information acquired with the two-dimensional image and the second distance information to detect a three-dimensional shape or volume occupied for example by a person present in the monitoring area and can compute the relative position of the person to the camera by combining the two-dimensional image with the second distance information.

According to the invention, the controller is further configured to compare the first and the second position information and to identify the at least one person at the position represented by the first position information with the at least one person at the position represented by the second position information when the first position information matches the second position information.

In other words, the controller is thus configured to acquire a first position (represented by first position information) of a person e.g. present in the monitoring area based on the system of first transceivers and second transceivers (for example the RF system including RF listeners and RF badges) and to acquire identification information of the person e.g. provided with the second transceiver. The controller is further adapted to acquire a second position (represented by second position information) of a person recognized as such within a two-dimensional image. This second position is acquired based on the second distance information. When the position determined via the system of first and second transceivers matches the position determined for the person recognized as such in the two-dimensional image, the controller may e.g. assign the identification information acquired to the system of first and second transceivers to the person recognized as such in the two-dimensional image.

In a preferred embodiment, the controller is further configured to determine a two-dimensional shape of the at least one person imaged by the imaging means within the two-dimensional image, and to determine a three-dimensional shape or volume of the at least one person imaged within the two-dimensional image based on the two-dimensional shape of the person. For example, in a preferred embodiment, the controller is configured to perform image processing of the two-dimensional image to recognize a two-dimensional shape of a person within the two-dimensional image. Based on this two-dimensional shape, the controller can then preferably be further configured to calculate a three-dimensional shape or volume, i.e. a three-dimensional model of the geometry of the person. The three-dimensional model may be an actual three-dimensional surface representing a proximate outer surface of the person or an estimated skeleton of the person. Such calculation may preferably be based on predefined image processing libraries relating two-dimensional shapes of humans to typical three-dimensional human shapes or typical human skeletons.

Preferably, the second distance information represents distances between portions of the at least one person and the imaging means, the controller being configured to determine a three-dimensional shape of the at least one person based the second distance information. The controller may thus be further or alternatively be configured to determine a three-dimensional shape or volume based on distances (second distance information) between portions of the at least one person (corresponding to respective image portions of the two-dimensional image acquired by the imaging means) and the imaging means.

In a preferred embodiment, the controller is further configured to determine a three-dimensional shape of a further person and a relative distance between the at least one person and the further person based on the corresponding three-dimensional shapes, and to determine that the first person is in contact with the second person when the relative distance is smaller than a predetermined threshold. The relative distance may be a minimum distance between portions of the three-dimensional shapes of the first and the second persons. Portions may be body parts or differently defined portions of each three dimensional shape. Preferably, the relative distance is the distance between a hand of the first person, e.g. the HCW, and the body, i.e. the three-dimensional shape, of the second person, e.g. the patient. To this end, the controller may preferably be configured to recognize a hand of the first person from the two-dimensional shape or the three-dimensional shape of the first person.

In addition or alternatively, in the case that the three-dimensional model is a proximate human skeleton, the controller may be configured to recognize joints of the persons' bodies as respective portions. For example, such distance may be the distance between a hand joint such as a finger joint or a wrist joint of the first person and a hip joint or knee joint of the further person. Thus, preferably, the relative distance is the distance between a joint of a hand or a wrist joint of the first person and a joint of the further person.

Thereby, the threshold may be set in advance and can be adjusted in accordance with the requirements of the particular system such as in accordance with the resolution of the two-dimensional image and the like. In a preferred embodiment, the controller is further configured to determine a two-dimensional shape of an object different from a person within the two-dimensional image, to determine a three dimensional shape of the object and to determine that the at least one person is in contact with the object when a relative distance between the at least one person and the object is below a predefined threshold. Such objects may be objects e.g. surrounding a patient such as a nightstand, a bed or the like.

In a preferred embodiment, the controller further comprises a dispenser signal receiving means configured to receive a dispenser signal transmitted from a cleaning agent dispenser detector provided at a cleaning agent dispenser when a person uses the cleaning agent dispenser. Thereby, a cleaning agent may be soap or a sterilizing agent as it is typically used for example in medical facilities such as hospitals.

Preferably, the controller is further configured to determine a relative distance between the first person based on the first position information and a predetermined position of the cleaning agent dispenser. Thus, the controller is configured to determine a relative distance between the first person based on the information received from the system of first and second transceivers. Alternatively, in a different preferred embodiment, the controller is configured to determine a relative distance between the first person based on a communication between the second transceiver provided at the first person and a dispenser transceiver, i.e. a transceiver similar to the first or second transceiver, provided at the dispenser. For example, when the distance between the second transceiver of the first person and the dispenser transceiver of the dispenser is below a predefined threshold, one of the transceivers may transmit a signal, the controller being configured to receive the signal thus determining the relative distance between the first person and the cleaning agent dispenser.

Preferably, the controller is configured to determine that the first person has used the cleaning agent dispenser, when the dispenser signal receiving means receives the dispenser signal and the relative distance between the first person and a position of the cleaning agent dispenser is below a predetermined threshold. Thus, when the controller receives the dispenser signal and determines that the first person (preferably identified by identification information received from the first transceiver or the second transceiver provided at the first person) is close to the cleaning agent dispenser (the relative distance between said predetermined thresholds), the controller determines that the first person has used the cleaning agent dispenser.

Preferably, the controller is further configured to determine that the first person has entered a monitoring area at a first timing based on a signal received from the at least one first transceiver, and is configured to determine that the first person is in contact with the second person at a second timing, and is configured to generate an output when determining that in a time interval between the first timing and the second timing the controller has not determined that the first person has used the cleaning agent dispenser.

In other words, when the person enters a monitoring area such as a room or an area defined e.g. by reception quality of the used first and second transceivers, or into a differently defined monitoring area, the controller is configured to determine this entering to be performed at a first timing. For example, when a HCW provided with a second transceiver, e.g. an RF tag, enters a patient room, entering the room may be determined by the controller via communication between the at least one first transceiver and the second transceiver provided at the first person. The second transceiver may e.g. send a signal to the at least one first transceiver, e.g. an RF listener, which in turn may send a signal to the controller. Alternatively, the first transceiver may broadcast signals at given timings which are received by a second transceiver when a person enters the monitoring area. The second transceiver may then send a response signal which is transferred to the controller via the first transceiver. The first timing is in these cases the time point when the signal is received by the controller.

Preferably, the controller is further configured to monitor the relative distance between the first person and the second person, and is configured to cause an alert when at a third timing the relative distance between the first person and the second person is below a predefined threshold and in a time interval between the first timing and the third timing the controller has not determined that the first person has used the cleaning agent dispenser.

In other words, the controller is adapted to monitor e.g. the first Moment of the "Five Moments" defined by the WHO. The controller is thus adapted to monitor e.g. actions of a first person such as a HCW, within a monitoring area such as a patient room and is adapted to determine contact between said first person and a second person such as a patient. In addition, the controller is adapted to monitor if in advance to such contact the first person, e.g. the HCW, has used the cleaning agent dispenser. The controller may further be adapted to monitor the further Moments as defined by the WHO, i.e. further actions of the HCW cleaning his/her hands after touching the patient, after touching the patient surroundings, etc. Based on such determination, it is thus possible to monitor compliance with a hygiene protocol such as the "Five Moments" as defined by the WHO. Thus, preferably, the controller is a controller for a hygiene compliance monitoring system.

The present invention further provides a system, preferably for a hygiene compliance monitoring, more preferably for a hand hygiene compliance monitoring system, whereby the system comprises the controller as described above, the at least one first transceiver, the second transceiver, and the imaging means.

Preferably, the hygiene compliance monitoring system further comprises a second distance information acquiring means for acquiring the second distance information, the second distance information acquiring means and the imaging means being comprised by a 3D-camera. Thereby, a 3D-camera can be a combination of a two-dimensional digital camera and a means to determine the distance between objects imaged by the camera and the camera such as a two-dimensional time of flight detector, whereby a distance between an object imaged by a camera is measured by measuring the time between sending a light signal from the camera and reception of the signal reflected from the imaged object. This method allows to perform measurements for individual image portions of a two-dimensional image such that a "depth image" as described above can be obtained with portions of the depth image representing distances for corresponding image portions of the two-dimensional image.

In a preferred embodiment, the monitoring area is a room, preferably a patient room in a hospital, and the at least one first transceiver is provided at a wall or door of the room.

Further, preferably, the first and second transceivers are radio frequency (RF) transceivers. In an alternative preferred embodiment, the first and second transceivers are infrared (IR) or ultrasound transceivers.

Further, according to the invention a method preferably for hygiene compliance monitoring, more preferably for hand hygiene compliance monitoring, is provided which comprises the following steps:
receive first distance information acquired by at least one first transceiver representing a distance between the at least one first transceiver and at least one second transceiver provided at at least one person;
determine first position information representing a position of the at least one person based on the first distance information;
receive person identification information from a person ID transceiver for identifying the at least one person;
receive data of a two-dimensional image acquired by an imaging means, and
receive second distance information representing at least one distance between the at least one person and the imaging means, whereby the at least one person is imaged by the imaging means,
determine second position information relating to the position of the at least one person imaged by the imaging means based on the second distance information;
compare the first and the second position information
identify the at least one person at the position represented by the first position information with the at least one person at the position represented by the second position information when the first position information matches the second position information.

### 4. Description of the preferred embodiments

Fig. 1 shows a schematic top view of a patient room in a hospital;
Fig. 2 shows a further top view of the patient room in the hospital;
Fig. 3 illustrates the cleaning agent dispenser;
Fig. 4 illustrates images acquired by the three-dimensional imaging means and the second distance information acquiring means;
Fig. 5 is a schematic illustration of the components of the hygiene compliance monitoring system; and
Fig. 6 illustrates the actions to be performed by the hygiene compliance monitoring system.

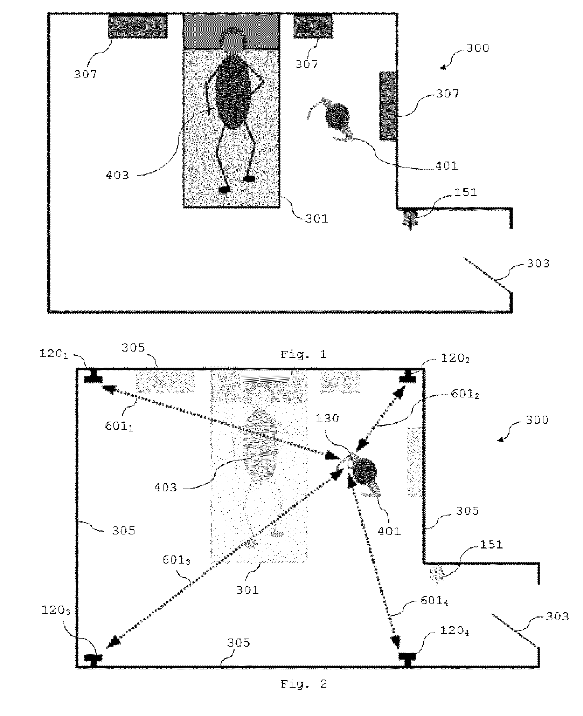

Fig. 1 shows a schematic top view of a patient room 300 in a hospital with a patient 403 on a bed and a health care worker (HCW) 401 present in the room, i.e. the monitoring area. Further, patient surroundings 307 are schematically illustrated which may be nightstands, dining tables, wardrobes or the like. In addition, a cleaning agent dispenser 151 is illustrated in proximity to the entrance door 303 such that a person entering or exiting the room 300 can use the cleaning agent to wash his or her hands.

Fig. 2 shows the patient room 300 of Fig. 1 further being provided with first transceivers 120₁, 120₂, 120₃ and 120₄ provided at predefined positions, i.e. at respective walls 305 of the patient room 300. In the preferred embodiment, the first transceivers are RF listeners adapted to send and receive RF signals to and from a corresponding second transceiver which in the preferred embodiment is an RF tag 130 provided at the HCW 401 or the patient 403. The RF listeners 120₁, 120₂, 120₃, and 120₄ are adapted to calculate a distance between the respective RF listener 120₁, 120₂, 120₃ and 120₄ and the RF tag 130 based on the time of flight of a signal communicated with the RF tag 130 along the dashed arrows 601₁, 601₂, 601₃ and 601₄ shown in Fig. 2. As the skilled person will understand, based on these four distances it is possible to determine the position of the HCW as indicated by the position of the RF badge 130 using a suitable algorithm such as trilateration, multi-lateration or the like. Thus, the relative distance between each of the RF listeners and the RF tags 130 is provided to the controller (not shown) as first distance information and the controller is adapted to calculate a position (first position information) of the HCW based on this first distance information. In addition, the RF tag has person identification information which can be acquired by the RF listener and send to the controller to determine the identity of the person present at the position represented by the first position information.

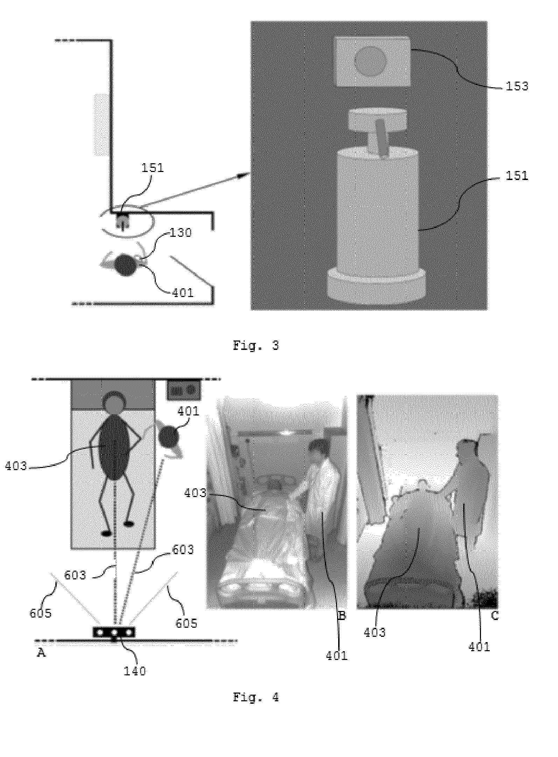

Fig. 3 illustrates the cleaning agent dispenser 151 in a different view (right part of Fig. 3). The cleaning agent dispenser is provided with a cleaning agent dispenser detector (not shown) which serves to send a signal to the controller when the HCW uses the dispenser. The controller additionally determines that the relative distance between the HCW 401 and the dispenser 151 is below a predefined threshold. This may be determined based on the position of the HCW 401 determined using the RF listeners 120₁, 120₂, 120₃ and 120₄ and a known position of the dispenser 151. In the shown embodiment, the relative distance is determined based on communication between the shown dispenser transceiver 153 provided at the dispenser and the RF badge 130. The controller can thus determine that the HCW has washed his or her hands.

Fig. 4 illustrates the use of the imaging means and the second distance information acquiring means which in the shown embodiment are comprised by a 3D-camera 140. The 3D-camera 140 is adapted to acquire a two-dimensional image as illustrated in part B of Fig. 4, whereby the corresponding field of view is indicated by dotted lines 605 in part A of the figure. In the shown example the image is an infrared image. However, a visible image similarly could be employed. The 3D-camera 140 is further adapted to acquire a corresponding depth image as illustrated in part C of Fig. 4 wherein for each image portion the distance between objects imaged in the two-dimensional image with respect to the camera 140 are indicated using a grey scale coding. Such distances are exemplarily indicated by the dashed lines 603 in part A of the figure. As can be taken from Fig. 4, the distance of the object imaged in a portion of the two-dimensional image with respect to the 3D-camera 140 is encoded in a corresponding portion of the depth image. In other words, parts B and C of Fig. 4 illustrate a one-to-one correspondence between the two-dimensional image of part B and the depth image of part C.

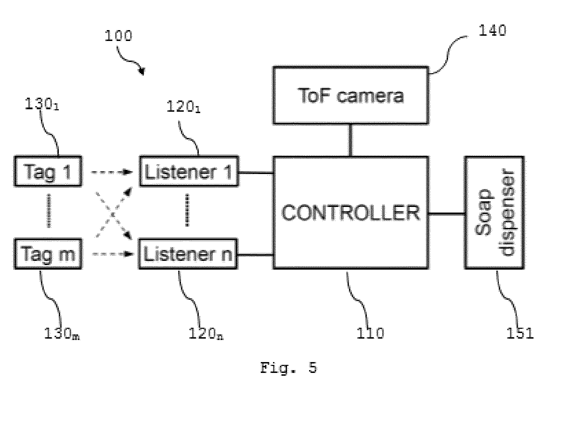

Fig. 5 schematically illustrates components of the hygiene compliance monitoring system 100. As illustrated, tags 130₁ to 130ₘ can be provided at m persons present in the monitoring area, for example the patient room. In addition, n listeners 120₁ to 120ₙ, i.e. receivers, are provided which exchange or communicate signals, for example RF signals with the m tags. Based on this communication, each listener can determine the relative distance between said listener and each of the RF tags and these relative positions are supplied as first distance information to the controller 110. In addition, each tag is provided with person identification information for identifying the person provided with the tag which is sent to the listener, this person identification information is similarly provided to the controller 110. Based on the first distance information, the controller can determine the position of each person within the monitoring area based on this first distance information and in addition knows the person identification information corresponding to each position.

In addition, a time of flight camera 140 is shown as an example of a 3D-camera being connected to the controller 110. Alternatively, different 3D-cameras such as stereoscopic cameras may also be used. Based on image processing of images acquired by the time of flight camera and on second distance information acquired from said time of flight camera, the controller can assign person identification information received from the system of RF tags and listeners to a person recognized as such in an image received from the time of flight camera when the first position information matches the second position information determined based on second distance information received from the time of flight camera.

In addition, a soap dispenser 151 is illustrated as an example of a cleaning agent dispenser connected to the controller to notify the controller when the soap dispenser is used by sending a cleaning agent dispenser signal.

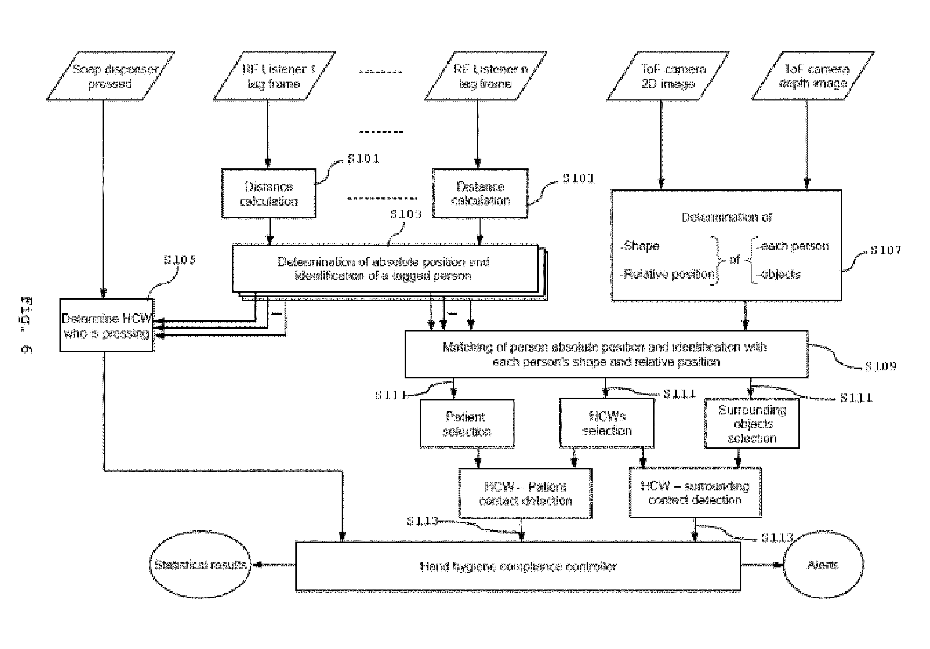

Fig. 6 is a flow chart illustrating steps performed by the hygiene compliance monitoring system. As illustrated in this flow chart, based on signals communicated with n RF listeners and corresponding RF tags, distances between the RF listeners and the RF tags can be calculated (S101). Based on these distances (first distance information) an absolute position of the person provided with a tag can be determined in addition to its identity (S103). When this person is near to the soap dispenser and it is determined that the soap dispenser is pressed, the controller is adapted to determine the HCW who is pressing the soap dispenser (S105).

By analyzing the two-dimensional image acquired by the time of flight camera and the depth image acquired by said camera, the controller determines three-dimensional shape or volume and relative position with respect to the camera for each person and object present in the monitoring area (S107). The controller then compares the position acquired via the system of RF listeners and RF tags with the position acquired based on the relative position of a person or object with respect to the camera and assigns person identification information to said person (S109).

Taking into account the identification information, the controller can select a patient, HCWs and surrounding objects present in the two-dimensional image (S111). The controller can then determine if HCW and patient are in contact or if HCW is in contact with surrounding objects (S113). Through monitoring these actions, it becomes possible that the controller automatically outputs statistical results monitoring the above steps over a predetermined time and in addition it becomes possible to output alerts, when for example it is determined that a HCW is in contact with a patient having not previously washed his or her hands.

## Claims

1. Controller (110) comprising:
first distance information receiving means configured to receive first distance information acquired by at least one first transceiver (120₁...120ₙ) representing a distance (601₁...601ₙ) between the at least one first transceiver (120₁...120ₙ) and at least one second transceiver (130₁...130ₘ) provided at at least one person (401, 403), the controller (110) being configured to determine first position information representing a position of the at least one person (401, 403) based on the first distance information;
person identification information receiving means configured to receive person identification information from a person ID transceiver (130₁...130ₘ) for identifying the at least one person (401, 403);
two-dimensional image data receiving means configured to receive data of a two-dimensional image (Fig. 4B) acquired by an imaging means (140), and
second distance information receiving means configured to receive second distance information representing at least one distance (603) between the at least one person and the imaging means (140), the at least one person imaged by the imaging means (140),
the controller (110) being configured to determine second position information relating to the position of the at least one person (401, 403) imaged by the imaging means based on the second distance information;
the controller being configured to compare the first and the second position information and to identify the at least one person at the position represented by the first position information with the at least one person at the position represented by the second position information when the first position information matches the second position information.

2. Controller (110) according to claim 1, further configured to determine a two-dimensional shape of the at least one person (401, 403) imaged by the imaging means (140) within the two-dimensional image, and to determine a three-dimensional shape of the at least one person (401, 403) imaged within the two-dimensional image based on the two-dimensional shape of the person (401, 403).

3. Controller (110) according to any one of claims 1 or 2, whereby the second distance information represents distances between portions of the at least one person and the imaging means (140), the controller being configured to determine a three-dimensional shape of the at least one person (401, 403) based on the second distance information.

4. Controller (110) according to any one of the preceding claims, further configured to determine a three-dimensional shape of a further person and a relative distance between the at least one person and the further person based on the corresponding three-dimensional shapes, and to determine that the first person (401, 403) is in contact with the second person (401, 403), when the relative distance is smaller than a predetermined threshold.

5. Controller (110) according to any one of the preceding claims further configured to determine a two-dimensional shape of an object different from a person within the two-dimensional image, to determine a three dimensional shape of the object and to determine that the at least one person is in contact with the object when a relative distance between the at least one person and the object is below a predefined threshold.

6. Controller (110) according to any one of the preceding claims, whereby the second distance information is a two-dimensional array (Fig. 4C) of distance information elements, and the data of the two-dimensional image (Fig. 4B) is a two-dimensional array of image information elements, whereby each of the distance information elements has a one-to-one relation with a corresponding one of the image information elements and represents a distance between an object imaged within an image information element and the imaging means.

7. Controller (110) according to any one of the preceding claims, further comprising a dispenser signal receiving means configured to receive a dispenser signal transmitted from a cleaning agent dispenser detector (153) provided at a cleaning agent dispenser (151) when a person (401, 403) uses the cleaning agent dispenser (151),
the controller (110) further being configured to determine a relative distance between the first person (401, 403) based on the first position information and a predetermined position of the cleaning agent dispenser (151), and
configured to determine that the first person (401, 403) has used the cleaning agent dispenser (151), when the dispenser signal receiving means receives the dispenser signal and the relative distance between the first person (401, 403), based on the first position information and a predetermined position of the cleaning agent dispenser is below a predetermined threshold.

8. Controller (110) according to any one of the preceding claims, further configured to determine that the first person (401, 403) has entered a monitoring area (300) at a first timing based on a signal received from the at least one first transceiver (120₁...120ₙ), and is configured to determine that the first person (401, 403) is in contact with the second person (401, 403) at a second timing, and is configured to generate an output when determining that in a time interval between the first timing and the second timing the controller (110) has not determined that the first person (401, 403) has used the cleaning agent dispenser.

9. Controller (110) according to claim 8, the controller (110) further configured to monitor the relative distance between the first person (401, 403) and the second person (401, 403), and is configured to cause an alert when at a third timing the relative distance between the first person (401, 403) and the second person (401, 403) is below a predefined threshold and in a time interval between the first timing and the third timing the controller (110) has not determined that the first person (401, 403) has used the cleaning agent dispenser.

10. Controller according to any one of the preceding claims, the controller being for a hygiene compliance monitoring system (100).

11. System comprising the controller (110) according to any one of claims 1 to 10, the at least one first transceiver (120₁...120ₙ), the second transceiver (130₁...130ₘ), and the imaging means (140).

12. System (100) according to claim 11, further comprising a second distance information acquiring means for acquiring the second distance information, the second distance information acquiring means and the imaging means (140) being comprised by a 3D-camera.

13. System (100) according to any one of claims 11 or 12, or controller (110) according to any one of claims 1 to 10, whereby the at least one transceiver is provided at a predefined position within a monitoring area (300).

14. System (100) according to any one of claims 11 to 13, or controller (110) according to any one of claims 1 to 10 or 13, whereby the first distance information is calculated based on the time needed to communicate a signal between the first and the second transceiver (130₁...130ₘ).

15. Method comprising the following steps:
receive first distance information acquired by at least one first transceiver (120₁...120ₙ) representing a distance (601₁...601ₙ) between the at least one first transceiver (120₁...120ₙ) and at least one second transceiver (130₁...130ₘ) provided at at least one person (401, 403);
determine first position information representing a position of the at least one person (401, 403) based on the first distance information;
receive person identification information from a person ID transceiver (130₁...130ₘ) for identifying the at least one person (401, 403);
receive data of a two-dimensional image (401) acquired by an imaging means (140), and
receive second distance information representing at least one distance (603) between the at least one person and the imaging means (140), the at least one person imaged by the imaging means (140),
determine second position information relating to the position of the at least one person (401, 403) imaged by the imaging means based on the second distance information;
compare the first and the second position information
identify the at least one person at the position represented by the first position information with the at least one person at the position represented by the second position information when the first position information matches the second position information.
